# EUROPEAN PATENT APPLICATION

(11) **EP 3 593 665 A1**
(43) Date of publication of application: **15.01.2020**
(21) Application number: 18763407.6
(22) Date of filing: 02.03.2018
(51) Int. Cl.: A43B 17/00

(54) **CAPACITOR PRESSURE SENSING INSOLE AND OPERATION METHOD THEREOF**

(30) Priority: 09.03.2017 CN 201710137105
(71) Applicant: Qingyuan Global Technology Services Ltd., Qingyuan City, Guangdong 511800 (CN)
(72) Inventor: LUH, Yih-Ping, Taipei City 104 (TW)
(74) Representative: dompatent von Kreisler Selting Werner - Partnerschaft von Patent- und Rechtsanwälten mbB
(86) International application number: PCT/CN2018/077911
(87) International publication number: WO 2018/161860

(57) **Abstract**

A capacitive pressure detection insole is disclosed. The capacitive pressure detection insole includes a plurality of capacitive sensing nodes and an operating chip. When the capacitive pressure detection insole is subjected to a pressure provided by a foot of a user, the capacitive pressure detection insole uses the plurality of capacitive sensing nodes to sense a plurality of capacitance variations corresponding to a plurality of detection positions of the foot respectively. The operating chip receives the plurality of capacitance variations from the plurality of capacitive sensing nodes and obtains a pressure distribution information corresponding to the plurality of detection positions of the foot to determine a motion physiological status information of the foot of the user.

## Description

### BACKGROUND OF THE INVENTION

### 1. Field of the invention

The invention relates to an insole; in particular, to a capacitive pressure detection insole and an operating method thereof.

### 2. Description of the prior art

As the technology continues to evolve, the insole disposed in the shoe not only provides the conventional mat function, but also provides different functions such as heightening and shock absorption through different designs of thickness and material.

However, since the distribution of the pressure points at the sole of each user is different, if the insoles currently produced on the market are disposed in the shoes, the practical needs of each user cannot be met, resulting in that many users feel uncomfortable when wearing shoes walking or exercising, and the conventional insoles fail to make corresponding corrections for each user's foot problems, so that the user's foot problems cannot be improved or even worsened.

### SUMMARY OF THE INVENTION

Therefore, the invention provides a capacitive pressure detection insole and an operating method thereof to solve the above-mentioned problems of the prior arts.

A preferred embodiment of the invention is a capacitive pressure detection insole. In this embodiment, the capacitive pressure detection insole includes a plurality of capacitive sensing nodes and an operating chip. When the capacitive pressure detection insole is subjected to a pressure provided by a foot of a user, the capacitive pressure detection insole uses the plurality of capacitive sensing nodes to sense a plurality of capacitance variations corresponding to a plurality of detection positions of the foot respectively. The operating chip receives the plurality of capacitance variations from the plurality of capacitive sensing nodes and obtains a pressure distribution information corresponding to the plurality of detection positions of the foot to determine a motion physiological status information of the foot of the user.

In an embodiment, one of the capacitive sensing nodes is located at a cross between a capacitive yarn and a conductive yarn, and two electrically conductive coatings are formed on a surface of the capacitive yarn.

In an embodiment, when the capacitive pressure detection insole is subjected to the pressure provided by the foot of the user, the capacitive yarn is crushed by the pressure to cause charge dispersion, under a condition that a distance between the two electrically conductive coatings is constant, the capacitance is changed due to the charge density becomes smaller, so that the plurality of capacitive sensing nodes sense the plurality of capacitance variations.

In an embodiment, the capacitive yarn and the conductive yarn are wrapped or interlaced with each other.

In an embodiment, the capacitive yarn and the conductive yarn are disposed under a layer of thermoplastic polyester elastomer (TPEE) or between two layers of TPEE.

In an embodiment, the operating chip is a blue-tooth chip or an internet of thing (IoT) chip embedded in the capacitive pressure detection insole.

In an embodiment, when the operating chip receives the plurality of capacitance variations, the operating chip screens and converts the plurality of capacitance variations to reduce an amount of data of the plurality of capacitance variations.

Another preferred embodiment of the invention is a capacitive pressure detection insole operating method. In this embodiment, the capacitive pressure detection insole operating method is used to operate a capacitive pressure detection insole. The capacitive pressure detection insole includes a plurality of capacitive sensing nodes and an operating chip. The capacitive pressure detection insole operating method includes steps of: when the capacitive pressure detection insole is subjected to a pressure provided by a foot of a user, using the plurality of capacitive sensing nodes to sense a plurality of capacitance variations corresponding to a plurality of detection positions of the foot respectively; and the operating chip receiving the plurality of capacitance variations from the plurality of capacitive sensing nodes and obtaining a pressure distribution information corresponding to the plurality of detection positions of the foot to determine a motion physiological status information of the foot of the user.

Compared to the prior art, the capacitive pressure detection insole and the operating method thereof according to the invention can sense different pressure points on the sole of each user respectively through a plurality of capacitive sensing nodes disposed at the positions where the capacitive yarn and the conductive yarn are interdigitated respectively to obtain the motion physiological status information about the foot of each user, and further designing a customized orthopedic insole for each user's foot problem, thereby effectively improving the comfort of the user when wearing the shoe and the user's foot problem can be significantly improved.

The advantage and spirit of the invention may be understood by the following detailed descriptions together with the appended drawings.

### BRIEF DESCRIPTION OF THE APPENDED DRAWINGS

FIG. 1 illustrates a schematic diagram of the capacitive pressure detection insole 2 disposed in the shoe 1 in the invention.
FIG. 2 illustrates a schematic diagram of the capacitive pressure detection insole 2 having a plurality of capacitive sensing nodes N in the invention.
FIG. 3 illustrates a schematic diagram of the plurality of capacitive sensing nodes N in the capacitive pressure detection insole 2 disposed under the TPEE layer TP and the operating chip CH embedded in the capacitive pressure detection insole 2 in the invention.
FIG. 4 illustrates a schematic diagram showing that when the capacitive pressure detection insole 2 is subjected to the pressure provided by the user's foot FT, the operating chip CH receives a plurality of capacitance variations corresponding to a plurality of detection positions P1, P2, P3, ... of the foot FT sensed by the plurality of capacitive sensing nodes N1, N2, N3, ... respectively and connects to the cloud database DB or mobile communication device MB via the network NET.
FIG. 5 illustrates a schematic diagram showing that the capacitive yarn and the conductive yarn are wrapped or interlaced with each other.
FIG. 6 illustrates a flowchart of the capacitive pressure detection insole operating method in the invention.

### DETAILED DESCRIPTION OF THE INVENTION

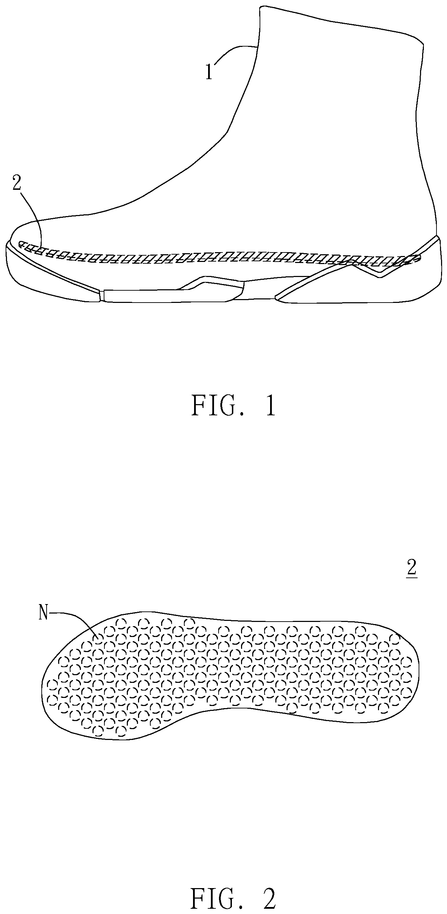

A preferred embodiment of the invention is a capacitive pressure detection insole. As shown in FIG. 1, the capacitive pressure detection insole 2 is disposed in the shoe 1. When the user puts on the shoe 1, the user's foot provides a pressure to the capacitive pressure detection insole 2. At this time, the capacitive pressure detection insole 2 can sense the capacitance variations corresponding to different detection positions of the foot through a plurality of capacitive sensing nodes N (as shown in FIG. 2) to obtain the motion physiological status information about the foot of the user.

In addition, the invention can further determine the respective foot problems of different users according to the motion physiological status information about the foot of the different users, so as to design the customized insole for each user according to the foot problems of different users respectively. Therefore, the user can feel comfortable when wearing shoes and the user's foot problems can be significantly improved to achieve corrective effects.

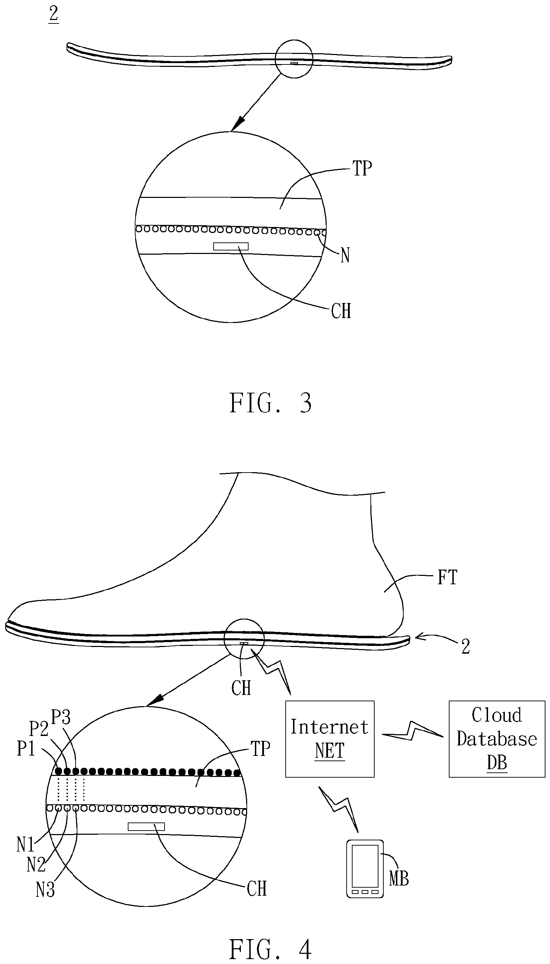

Please refer to FIG. 3 and FIG. 4. FIG. 3 illustrates a schematic diagram that the capacitive pressure detection insole 2 is not subjected to the pressure provided by the user's foot in the invention; FIG. 4 illustrates a schematic diagram that the capacitive pressure detection insole 2 is subjected to the pressure provided by the user's foot in the invention.

As shown in FIG. 3 and FIG. 4, the capacitive pressure detection insole 2 can include a thermoplastic polyester elastomer (TPEE) layer TP, a plurality of capacitive sensing nodes N and an operating chip CH. The plurality of capacitive sensing nodes N is disposed under the thermoplastic polyester elastomer layer TP and the operating chip CH is embedded in the capacitive pressure detection insole 2.

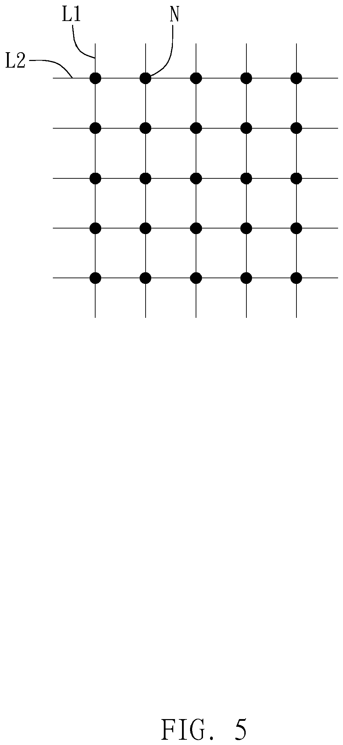

In practical applications, as shown in FIG. 5, the capacitive yarn L1 and the conductive yarn L2 are wrapped or interlaced with each other, and the capacitive yarn L1 and the conductive yarn L2 can be disposed under the thermoplastic polyester elastomer layer TP, and the plurality of capacitive sensing nodes N is located at the intersections of the capacitive yarn L1 and the conductive yarn L2, but not limited to this. In fact, the capacitive yarn L1 and the conductive yarn L2 can also be disposed between the two thermoplastic polyester elastomer layers TP.

It should be noted that two electrically conductive coatings are formed on a surface of the capacitive yarn L1 and a capacitor is formed when there is a charge distribution between the two electrically conductive coatings. When the capacitive pressure detection insole 2 has not been subjected to the pressure provided by the user's foot FT, the charge distribution between the two electrically conductive coatings will be denser, that is, the charge density is higher; when the capacitive pressure detection insole 2 is subjected to the pressure provided by the user's foot FT, the capacitive yarn L1 is crushed by the pressure, so that the charge distribution between the two electrically conductive coatings will become more dispersed. Under the condition that the distance between the two electrically conductive coatings is constant, the capacitance value changes due to the decrease of the charge density, and the plurality of capacitive sensing nodes N located at the intersections of the capacitive yarn L1 and the conductive yarn L2 respectively senses a plurality of capacitance variations.

For example, as shown in FIG. 4, when the user's foot FT steps on the capacitive pressure detection insole 2, the positions of the plurality of capacitive sensing nodes N1, N2, N3, ... of the capacitive pressure detection insole 2 respectively correspond to the plurality of detection positions P1, P2, P3, ... of the foot FT. Therefore, the plurality of capacitive sensing nodes N1, N2, N3, ... of the capacitive pressure detection insole 2 correspondingly sense the plurality of capacitance variations on the plurality of detection positions P1, P2, P3, ... of the foot FT.

When the plurality of capacitive sensing nodes N1, N2, N3, ... respectively senses the plurality of capacitance variations corresponding to the plurality of detection positions P1, P2, P3, ... of the foot FT, the operating chip CH will receive the plurality of capacitance variations from the capacitance sensing nodes N1, N2, N3, ... and obtain a pressure distribution information corresponding to the plurality of detection positions P1, P2, P3, ... of the foot FT according to the plurality of capacitance variations.

In practical applications, the operating chip CH can store the received data (i.e., the plurality of capacitance variations corresponding to the plurality of detection positions P1, P2, P3, ... of the foot FT respectively). The operating chip CH can also perform operation programs such as screening and conversion on the received data to reduce the amount of data. In addition, the operating chip CH can determine the motion physiological status of the user's foot based on the pressure distribution information corresponding to the plurality of detection positions P1, P2, P3, ... of the foot FT of the user.

It should be noted that the operating chip CH used in the capacitive pressure detection insole 2 of the invention can be a bluetooth chip or an internet of thing (IoT) chip, and it can be embedded in any position within the capacitive pressure detection insole 2, but not limited to this.

In practical applications, as shown in FIG. 4, the operating chip CH can also be connected to the network NET and upload the pressure distribution information and/or the motion physiology status information of the user's foot FT correspond to the plurality of detection positions P1, P2, P3, ... of the foot FT to the cloud database DB for reference for subsequent applications through the network NET.

For example, the insole manufacturer can obtain the motion physiological status information of the foot FT of the user A through the cloud database DB and judge the user's foot problem according to the motion physiological status information. Then, the insole manufacturer can customize the customized correction insole for the user A and set it in the shoe. When the user A wears the shoe with the customized correction insole and walks for a period of time, the user's foot problem should be significantly improved.

In addition, the user A can also operate the application (APP) of the mobile communication device (such as a smart phone) MB to connect to the operating chip CH or the cloud database DB through the network NET, so as to obtain the motion physiological status information of the foot FT of the user A at any time.

Another preferred embodiment of the invention is a capacitive pressure detection insole operating method. In this embodiment, the capacitive pressure detection insole operating method is used to operate a capacitive pressure detection insole. The capacitive pressure detection insole includes a plurality of capacitive sensing nodes and an operating chip. The plurality of capacitive sensing nodes is located at a cross between the capacitive yarn and the conductive yarn, and two electrically conductive coatings are formed on a surface of the capacitive yarn. The capacitive yarn and the conductive yarn are wrapped or interlaced with each other under a layer of thermoplastic polyester elastomer (TPEE) or the capacitive yarn and the conductive yarn are wrapped or interlaced with each other between two layers of TPEE to form the capacitive pressure detection insole. the operating chip is a blue-tooth chip or an internet of thing (IoT) chip embedded in the capacitive pressure detection insole.

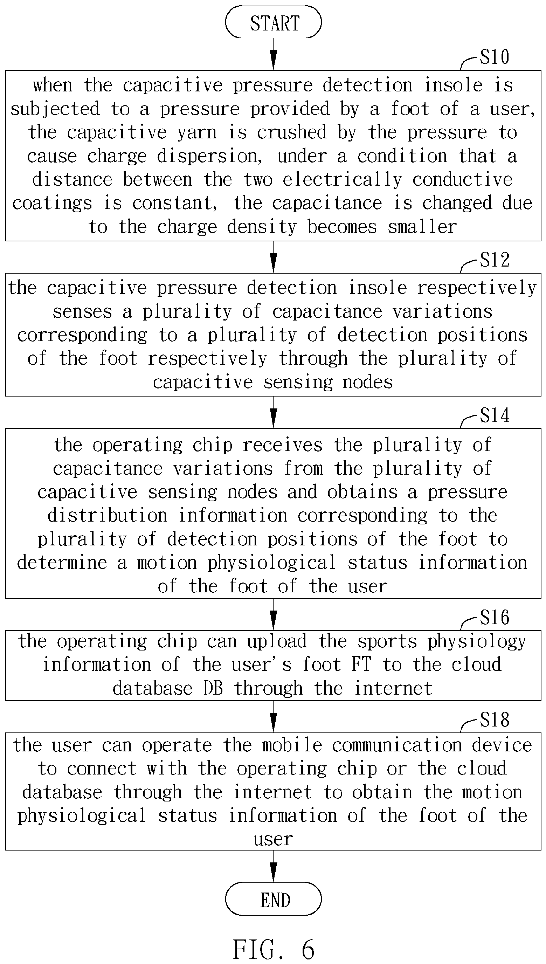

Please refer to FIG. 6. FIG. 6 illustrates a flowchart of the capacitive pressure detection insole operating method in the invention. As shown in FIG. 6, the capacitive pressure detection insole operating method can include steps of:
Step S10: when the capacitive pressure detection insole is subjected to a pressure provided by a foot of a user, the capacitive yarn is crushed by the pressure to cause charge dispersion, under a condition that a distance between the two electrically conductive coatings is constant, the capacitance is changed due to the charge density becomes smaller.
Step S12: the capacitive pressure detection insole respectively senses a plurality of capacitance variations corresponding to a plurality of detection positions of the foot respectively through the plurality of capacitive sensing nodes.
Step S14: the operating chip receives the plurality of capacitance variations from the plurality of capacitive sensing nodes and obtains a pressure distribution information corresponding to the plurality of detection positions of the foot to determine a motion physiological status information of the foot of the user. In fact, the operating chip can also filter and convert the plurality of capacitance variations to reduce the amount of data of the plurality of capacitance variations.
Step S16: the operating chip can upload the sports physiology information of the user's foot FT to the cloud database DB through the internet.
Step S18: the user can operate the mobile communication device to connect with the operating chip or the cloud database through the internet to obtain the motion physiological status information of the foot of the user.

Compared to the prior art, the capacitive pressure detection insole and the operating method thereof according to the invention can sense different pressure points on the sole of each user respectively through a plurality of capacitive sensing nodes disposed at the positions where the capacitive yarn and the conductive yarn are interdigitated respectively to obtain the motion physiological status information about the foot of each user, and further designing a customized orthopedic insole for each user's foot problem, thereby effectively improving the comfort of the user when wearing the shoe and the user's foot problem can be significantly improved.

With the example and explanations above, the features and spirits of the invention will be hopefully well described. Those skilled in the art will readily observe that numerous modifications and alterations of the device may be made while retaining the teaching of the invention. Accordingly, the above disclosure should be construed as limited only by the metes and bounds of the appended claims.

## Claims

1. A capacitive pressure detection insole, **characterized in that** the capacitive pressure detection insole comprises:
a plurality of capacitive sensing nodes, when the capacitive pressure detection insole is pressed by a foot of a user, the plurality of capacitive sensing nodes being configured to sense a plurality of capacitance variations corresponding to a plurality of detection positions of the foot respectively; and
an operating chip, configured to receive the plurality of capacitance variations from the plurality of capacitive sensing nodes and obtain a pressure distribution information corresponding to the plurality of detection positions of the foot to determine a motion physiological status information of the foot of the user.

2. The capacitive pressure detection insole of claim 1, **characterized in that** one of the capacitive sensing nodes is located at a cross between a capacitive yarn and a conductive yarn, and two electrically conductive coatings are formed on a surface of the capacitive yarn.

3. The capacitive pressure detection insole of claim 2, **characterized in that** when the capacitive pressure detection insole is subjected to a pressure provided by the foot of the user, the capacitive yarn is crushed by the pressure to cause charge dispersion, under a condition that a distance between the two electrically conductive coatings is constant, the capacitance is changed due to the charge density becomes smaller, so that the plurality of capacitive sensing nodes senses the plurality of capacitance variations.

4. The capacitive pressure detection insole of claim 2, **characterized in that** the capacitive yarn and the conductive yarn are wrapped or interlaced with each other.

5. The capacitive pressure detection insole of claim 4, **characterized in that** the capacitive yarn and the conductive yarn are disposed under a layer of thermoplastic polyester elastomer (TPEE) or between two layers of TPEE.

6. The capacitive pressure detection insole of claim 1, **characterized in that** the operating chip is a blue-tooth chip or an internet of thing (IoT) chip embedded in the capacitive pressure detection insole.

7. The capacitive pressure detection insole of claim 1, **characterized in that** the operating chip screens and converts the plurality of capacitance variations to reduce an amount of data of the plurality of capacitance variations.

8. A capacitive pressure detection insole operating method, **characterized in that** the capacitive pressure detection insole operating method is used for operating a capacitive pressure detection insole, the capacitive pressure detection insole comprises a plurality of capacitive sensing nodes and an operating chip, the capacitive pressure detection insole operating method comprises steps of:
when the capacitive pressure detection insole is subjected to a pressure provided by a foot of a user, using the plurality of capacitive sensing nodes to sense a plurality of capacitance variations corresponding to a plurality of detection positions of the foot respectively; and
the operating chip receiving the plurality of capacitance variations from the plurality of capacitive sensing nodes and obtaining a pressure distribution information corresponding to the plurality of detection positions of the foot to determine a motion physiological status information of the foot of the user.

9. The capacitive pressure detection insole operating method of claim 8, **characterized in that** one of the capacitive sensing nodes is located at a cross between a capacitive yarn and a conductive yarn, and two electrically conductive coatings are formed on a surface of the capacitive yarn.

10. The capacitive pressure detection insole operating method of claim 9, **characterized in that** when the capacitive pressure detection insole is subjected to a pressure provided by the foot of the user, the capacitive yarn is crushed by the pressure to cause charge dispersion, under a condition that a distance between the two electrically conductive coatings is constant, the capacitance is changed due to the charge density becomes smaller, so that the plurality of capacitive sensing nodes senses the plurality of capacitance variations.

11. The capacitive pressure detection insole operating method of claim 9, **characterized in that** the capacitive yarn and the conductive yarn are wrapped or interlaced with each other.

12. The capacitive pressure detection insole operating method of claim 11, **characterized in that** the capacitive yarn and the conductive yarn are disposed under a layer of thermoplastic polyester elastomer (TPEE) or between two layers ofTPEE.

13. The capacitive pressure detection insole operating method of claim 8, **characterized in that** the operating chip is a blue-tooth chip or an internet of thing (IoT) chip embedded in the capacitive pressure detection insole.

14. The capacitive pressure detection insole operating method of claim 8, **characterized in that** the operating chip screens and converts the plurality of capacitance variations to reduce an amount of data of the plurality of capacitance variations.
